# EUROPEAN PATENT APPLICATION

(11) **EP 4 817 593 A1**
(43) Date of publication of application: **07.10.2026**
(21) Application number: 25168073.2
(22) Date of filing: 02.04.2025
(51) Int. Cl.: A61K 8/34, A61K 8/41, A61K 8/42, A61K 8/44, A61K 8/60, A61Q 19/00, A61K 8/06

(54) **SKIN MOISTURISING COMPOSITIONS**

(71) Applicant: The Boots Company PLC, Nottingham NG2 3AA (GB)
(72) Inventor: JOHNSON, Mark, Nottingham, NG158 DQ (GB); WILLIAMS, Samantha, Nottingham, NG8 5AY (GB); PEREZ, Andrea, Leicester, LE11 2DX (GB); IRWIN, Robert, Nottingham, NG9 2AY (GB)

(57) **Abstract**

A skincare composition comprising (a) cationic emulsifier; and (b) at least 10 wt.% of a humectant, wherein the humectant comprises polyol.

## Description

### Field of the Invention

The invention relates to the field of skin care. More particularly, the invention relates to compositions, methods, and uses thereof for moisturisation of the skin.

### Background of the Invention

The moisture content of the skin is determined by a number of internal and external factors. The most important internal factors are the respiration of the skin (excretion of vapour), perspiration and the water binding constituents of the skin. Age, sex and body area can also have an effect. External factors that can influence the moisture content include environmental conditions (temperature, air humidity) and applied products (e.g. detergents, cosmetics and pharmaceuticals). The amount of moisture in the skin influences the formation of the hydro-lipid layer of the skin, which forms the basis of the skin's protective functions. Well hydrated skin has optimal elasticity and can potentially slow the signs of ageing.

Polyols are common cosmetic ingredients which are used as humectants, emollients, preservative boosters and texture enhancers. It is well known that polyols are used in moisturisers due to their ability to bind water when applied to the skin. Using a high level of polyol in a formulation is desirable to increase the moisturisation of a product but above a certain level the polyol can become sticky and tacky. Glycerin is a cost-effective polyol humectant which is particularly effective at skin moisturisation. It is typical for glycerin to be incorporated into emulsion moisturisers at levels up to approximately 8% before stickiness/tackiness can become apparent.

Existing solutions for moisturisation of the skin are usually based on anionic or non-ionic systems rather than a cationic system. Current (non-cationic) moisturisers are limited in the amount of humectant which can be added due to the negative sensorial attributes. Total humectant levels are therefore capped in the levels that can be used or extra ingredients need to be added to formulations to mitigate the stickiness/tackiness provided. These extra ingredients include common detacifiers, e.g. absorbent particles, emollients, silicones etc, raising cost and complexity.

Cationic products are common in haircare as conditioners but leave on moisturisers using cationic technology are not common. Some products exist on the market using cationic emulsifiers, but these use an old technology based on quaternary ammonium species (quats), which can be slightly irritating to the skin and eyes and persistent in the environment.

There is therefore a need for cationic systems for skin moisturising compositions that can incorporate higher levels of polyol humectants (e.g. glycerin) without the negative trade-offs on aesthetics from high levels of polyols and that can also provide long term moisturisation of the skin, improved skin mildness and can be formulated as a leave on skin moisturiser.

There is also a growing concern about the ecological impact and resistance to biodegradation of cationic compositions based on quaternary ammonium species (quats), and their potential high toxicity to the environment and aquatic life. Therefore, the is a need for products containing cationic emulsifiers with improved biodegradability and higher natural content.

The present invention provides skincare compositions comprising cationic emulsifiers that are able to incorporate high levels of polyol humectants (e.g. glycerin) without the associated stickiness or tackiness. The compositions of the present invention comprising new cationic technologies (e.g. esterquats and non-quats emulsifiers) also provide effective moisturisation of the skin and a powdery/non-greasy skin feel even with high oil, wax or glycerin blends. The use of these cationic systems also provides new compositions with improved biodegradation and lower toxicity to aquatic life, hence being more environmentally friendly compared to composition containing older quaternary surfactants.

### Summary of the Invention

The present invention therefore provides in a first aspect a skincare composition comprising (a) cationic emulsifier and (b) at least 10 wt.% of a humectant, wherein the humectant comprises polyol.

In a second aspect, the present invention provides a use of the skincare composition of the first aspect for topical cosmetic application to the skin of a subject.

In a third aspect, the present invention provides a use of the skincare composition of the first aspect for moisturising and/or hydrating the skin of a subject.

In a fourth aspect, the present invention provides a use of the skincare composition of the first aspect on skin as a non-therapeutic cosmetic treatment for any one or more of the following: improving skin moisture content, hydrating skin, improving the radiance of the skin, improving skin suppleness, reducing skin tightness, improving the healthy visual appearance of the skin, reducing skin dryness, improving the skin barrier function or any combination thereof.

In a fifth aspect, the present invention provides a method comprising applying the skincare composition of the first aspect to the skin of a subject.

In a sixth aspect, the present invention provides a cosmetic method for moisturising or hydrating a skin in need thereof, the method comprising topically administering the skincare composition of the first aspect of the invention to the skin of a subject.

In a seventh aspect, the present invention provides a cosmetic method of reducing skin dryness, the method comprising topically administering the skincare composition of the first aspect to the skin of a subject.

### Summary of the Figures

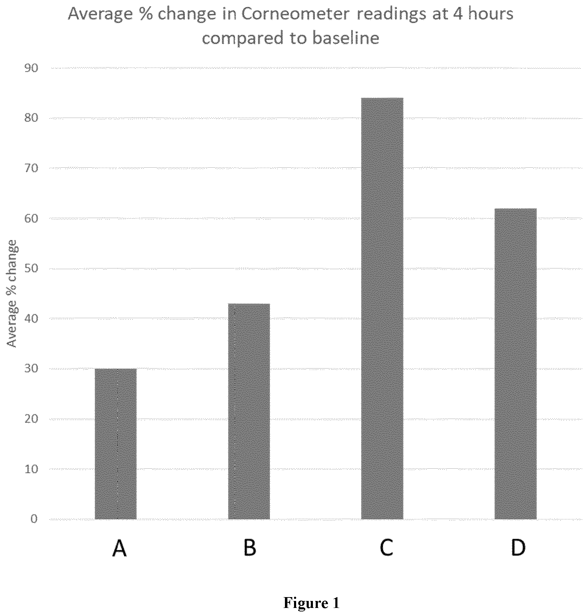

**Figure 1** shows a Corneometer study over 4 hours.

### Detailed Description of the Invention

The present invention relates to a skincare composition comprising (a) cationic emulsifier and (b) at least 10 wt.% of a humectant, wherein the humectant comprises a polyol.

The skincare composition of the present invention is a cosmetic composition. The skincare cosmetic composition of the invention may be facial skincare cosmetic composition. The skincare composition of the present invention does not include hair conditioning compositions.

Moisturisers are usually based on anionic or non-ionic systems rather than a cationic system. The composition of the present invention is based on a cationic system and comprises a cationic emulsifier.

The skincare composition of the present invention may be an emulsion. The skincare composition may be in the form of an oil-in-water emulsion. The composition of the present invention may be a cationic emulsion, such as for example a cationic oil-in-water emulsion. The composition of the present invention may be a moisturising composition or moisturiser.

The present inventors have surprisingly found that the use of cationic emulsifiers can form cationic compositions (e.g. cationic emulsions) with lamellar structures (lamellar liquid crystals). Said cationic compositions are able to incorporate high levels of humectants and oils without the associated stickiness or tackiness.

The composition of the present invention can surprisingly include levels of humectant such as glycerin above the maximum level typically used (up to 8 wt.%), for example above 10 wt.% (e.g. 10-22 wt.%), with a pleasant skin feel. Despite the high humectant content, the compositions of the present invention do not feel sticky, tacky or greasy on application to the skin and provide a powdery skin feel. This is presumably due to these ingredients being incorporated (shielded) into the lamellar structures of the emulsion. The compositions of the present invention have a consumer acceptable texture and have has shown improved moisturisation benefits compared to marketed cationic (quats) and non-cationic moisturisers.

The present inventors have also found that the compositions of the present invention comprising a cationic emulsifier are able to bind to the skin (skin has a negative charge) more efficiently and the lamellar structures can improve the penetration of the actives into the skin. This allows actives of the composition to work more effectively. The lamellar structures can also trap greater oil and water loads in the skin in a long-lasting stable format.

The skincare composition of the present invention comprising a cationic emulsifier also has improved sustainability credentials (natural content and biodegradability) compared to known composition containing quats.

In particular, topical application of the composition of the present invention is useful for improving skin moisture content and/or reducing skin drying and/or reducing transepidermal water loss (TEWL) from the skin's surface and providing short term and long term consistent moisturisation of the skin (including hydrating the skin). Topical application of the composition of the present invention is also useful for improving the healthy visual appearance and/or radiance of the skin.

The skincare composition of the present invention may comprise (a) a cationic emulsifier and (b) from 10 to 22 wt.% of a humectant (e.g. from 12-18 wt.%, based on the total weight of the composition), wherein the humectant comprises polyol (e.g. glycerin).

The skincare composition of the present invention may comprise (a) from 0.05% to 14 wt.% of a cationic emulsifier, and (b) from 10 to 22 wt.% (e.g. from 12-18 wt.%) of a humectant comprising polyol (e.g. glycerin).

The cationic emulsifier of the composition of the present invention may comprise an ester quaternary ammonium cationic emulsifier (esterquat cationic emulsifier) and/or a non-quaternary ammonium cationic emulsifier (non-quat cationic emulsifier).

The skincare composition of the present invention may comprise (a) a cationic emulsifier and (b) least 10 wt.% (e.g. from 10 to 22 wt.%, or from 12 to 18 wt.%) of a humectant comprising polyol, wherein the cationic emulsifier comprises an ester quaternary ammonium cationic emulsifier (esterquat cationic emulsifier) and/or a non-quaternary ammonium cationic emulsifier (non-quat cationic emulsifier); and wherein optionally the polyol comprises glycerin.

The skincare composition of the present invention may comprise (a) a cationic emulsifier, wherein the cationic emulsifier comprises an ester quaternary ammonium emulsifier (esterquat cationic emulsifier), an amino lipid emulsifier, an amidoamine emulsifier or combinations thereof; and (b) least 10 wt.% (e.g. from 10 to 22 wt.%, or from 12 to 18 wt.%) of a humectant comprising polyol; wherein optionally the polyol comprises glycerin.

The skincare composition of the present invention may comprise (a) cationic emulsifier, wherein the cationic emulsifier comprises distearoylisopropyl dimonium methosulfate, brassicamidopropyl dimethylamine, brassicyl valinate esylate, distearoylethyl dimonium chloride, bis-(isostearoyl/oleoyl isopropyl) dimonium methosulfate, cannabisamidopropyl dimethylamine, stearamidopropyl dimethylamine, brassicyl isoleucinate esylate or combinations thereof; and (b) least 10 wt.% (e.g. from 10-20 wt.%, or from 12-18 wt.%) of a humectant comprising polyol; wherein optionally the polyol comprises glycerin.

The skincare composition of the present invention may comprise (a) a cationic emulsifier and (b) from 10 to 22 wt.% of a humectant comprising polyol, wherein the polyol comprises glycerin, caprylyl glycol, ethylhexylglycerin, saccharide isomerate or combinations thereof.

The compositions of the present invention may be employed for purely cosmetic or skincare purposes. So the present invention further relates to a use of the skincare composition of the present invention for the topical application to the skin.

The skincare composition according to the present invention is used for application to the skin of a subject. The composition of the present invention may be used on any part of the skin, but preferably the composition is used on the face.

The skincare composition of the present invention may be used on skin for the purpose of any one of moisturising skin (including hydrating skin), improving skin moisture content, improving the radiance of the skin, improving skin suppleness, reducing skin tightness, improving the healthy visual appearance of the skin, nourishing skin, improving the skin barrier function, reducing skin dryness, or any combination thereof.

The present invention provides a use of the skincare composition of the present invention for moisturising and/or hydrating the skin of a subject.

The present invention provides a use of the composition of the invention as a non-therapeutic cosmetic treatment to improve the condition of the skin and/or improve skin moisture content and/or reduce skin dryness.

The use of the skincare composition according to the present invention may also improve skin barrier function when applied on a person's skin.

The use of the composition according to the present invention may reduce transepidermal water loss (TEWL) from the skin's surface by the topical application of the composition of the present invention.

The uses of the present invention comprise the skin composition described herein in all aspects and embodiments of the invention.

### Cationic emulsifier

The skincare compositions of the invention may comprise a cationic emulsifier.

The cationic emulsifiers used may be for use in water-in-oil or oil-in-water emulsions.

In the present invention, the cationic emulsifier does not comprise traditional quaternary ammonium compounds, such as centrimonium chloride (CTAC), behentrimonium chloride (BTAC) or dialkyldimethylammonium salts. The term "traditional quaternary ammonium compounds" does not include, imply or comprise ester-quats or quats with an ester bond in the structure of the cation. CTAC and BTAC are also classed as alkyl-quats, in the present invention, the cationic emulsifier is "alkyl quat-free".

The cationic emulsifier of the skincare compositions of the present invention may be an ester quaternary ammonium cationic emulsifier (esterquat cationic emulsifier) and/or a non-quaternary ammonium cationic emulsifier (non-quat cationic emulsifier). Non-quat or quat-free cationic emulsifiers do not contain a quaternary ammonium compound.

The cationic emulsifier may be an ester quaternary ammonium cationic emulsifier (esterquat), a cationic amino lipid emulsifier, an amidoamine cationic emulsifier or combinations thereof. Esterquats are quaternary ammonium compounds having an ester linkage.

The skincare composition of the present invention comprises a cationic emulsifier, wherein the cationic emulsifier may comprise ester quaternary ammonium compound (esterquat), amino lipid, amidoamine, or combinations thereof.

The cationic emulsifier may comprise beta hydroxy ammonium esters, amino ester lipids (or fatty acid amino esters), fatty acid amido alkyl di alkyl amine, or combinations thereof.

The cationic emulsifier of the skincare compositions of the present invention may comprise distearoylisopropyl dimonium methosulfate, brassicamidopropyl dimethylamine, brassicyl valinate esylate, distearoylethyl dimonium chloride, bis-(isostearoyl/oleoyl isopropyl) dimonium methosulfate, cannabisamidopropyl dimethylamine, stearamidopropyl dimethylamine, brassicyl isoleucinate esylate or combinations thereof. Brassicyl valinate esylate may be used as a mixture with brassica alcohol and brassica glycerides.

Suitable cationic emulsifiers may be commercially available under the trademark names TEGO^{®} Care Eco Cat MB from Evonik, AminoSensyl^{™} SC from Inolex, Procondition^{™} 22 from Inolex, Varisoft^{®} EQ 65 MB from Evonik, Varisoft^{®} EQ100 from Evonik, Procondition^{™} Sativa from Inolex, Emulsense^{™} HC from Inolex, Lexamine^{™} S-13 MB from Inolex.

The cationic emulsifier may be present in the skincare compositions of the present invention in an amount by weight of from 0.05% to 14%, such as from 0.1% to 14%, or from 0.15% to 14%, from 0.2% to 14%, from 0.3% to 14%, or from 0.05% to 0.12%, or from 0.1% to 12%, or from 0.15% to 12%, or from 0.2% to 12%, or from 0.3% to 12%, or from 0.05% to 10%, from 0.1% to 10%, or from 0.15% to 10%, or from 0.2% to 10%, or from 0.3% to 10%, or from 0.05% to 8%, or from 0.1% to 8%, or from 0.15% to 8%, or from 0.2% to 8%, or from 0.05% to 6%, or from 0.1% to 6%, or from 0.15% to 6%, or from 0.2% to 6%.

The cationic emulsifier may comprise amino ester lipids or fatty acid amino esters (e.g. brassicyl valinate esylate) which may be present in the composition in an amount by weight of from 1 to 14%, or from 1.5 to 14%, or from 2 to 14%, or from 1 to 12%, or from 1.5 to 12%, or from 2 to 12%, or from 1 to 10%, or from 1.5 to 10%, or from 2 to 10%, or from 1 to 8%, or from 1.5 to 8%, or from 2 to 8%.

The cationic emulsifier may comprise a amidoamine (e.g. brassicamidopropyl dimethylamine) which may be present in the composition in an amount by weight of from 0.05 to 6%, or from 0.05 to 5%, or from 0.0.5 to 4%, or 0.1 to 6%, or from 0.1 to 5.5%, or from 0.1 to 5%, or from 0.1 to 4.5%, or from 0.15 to 6%, or from 0.15 to 5.5%, or from 0.15 to 5%, or from 0.15 to 4.5%, or from 0.2 to 6%, or from 0.2 to 5.5%, or from 0.2 to 5%, or from 0.2 to 4.5%, or from 0.5 to 6%, or from 0.5 to 5.5%, or from 0.5 to 5%, or from 0.5 to 4.5%. or from 0.5 to 3%, or from 0.5 to 2%, or from 1 to 6%, or from 1 to 5.5%, or from 1 to 5%, or from 1 to 4%. or from 1 to 3%, or from 1 to 2%.

The cationic emulsifier may comprise an ester quaternary ammonium compound (esterquat) (e.g. distearoylisopropyl dimonium methosulfate) which may be present in the composition in an amount by weight of from 1 to 6%, or from 1 to 5.5%, or from 1 to 5%, or from 1 to 4.5%, or from 1.5 to 6%, or from 1.5 to 5.5%, or from 1.5 to 5%, or form 1.5 to 4.5%, or from 2 to 6%, or from 2 to 5.5%, or from 2 to 5%, or from 2 to 4.5%, or from 3 to 6%, or from 3 to 5.5%, or from 3 to 5%, or from 3 to 4.5%.

### Humectant

The skincare composition of the present invention comprises humectant comprising one or more polyols. The polyol may be a diol, a triol or a combination thereof. The composition may comprise a diol selected from the group consisting of pentylene glycol, decylene glycol, caprylyl glycol, butylene glycol, di-propylene glycol, ethylhexylglycerin, propanediol, hexenediol, propylene glycol, isoprene glycol, dipropylene glycol, pentylene glycol, hexylene glycol, polypropylene glycol, butylene glycol, polyethylene glycol, sorbitol, glucitol, mannitol, hydroxypropyl sorbitol, erythritol, threitol, pentaerythritol, xylitol and mixtures thereof. The composition may comprise a triol selected from the group consisting of hexanetriol, glycerol (glycerin), ethoxylated glycerin, propoxylated glycerin and mixtures thereof. The polyol may comprise maltitol, diglycerin, polyglycerin, saccharide isomerate and mixtures thereof.

Glycerin (CH₂OH-CHOH-CH₂OH) is also known as glycerol, glycerine, propane-1,2,3-triol, 1,2,3-propanetriol and 1,2,3-trihydroxypropane. The words "glycerol", "glycerin" and "glycerine" have been used interchangeably in the specification.

Saccharide isomerate consists mainly of glucose, fructose mannose and galactose. Saccharide isomerate mimics NMF (Natural Moisturising Factor) found in human skin and is derived from plant based glucose. The saccharide isomerate may be commercially available under the trademark name PENTAVITIN^{®} from DSM Nutritional Products Ltd.

The humectant in the composition of the present invention may comprise propylene glycol, also known as propanediol, butylene glycol, glycerin, saccharide isomerate, ethylhexylglycerin, panthenol, caprylyl glycol or combinations thereof.

The humectant in the composition of the present invention may comprise a diol and a triol. The humectant may comprise propylene glycol, also known as propanediol, butylene glycol, glycerin, ethylhexylglycerin, panthenol, and/or caprylyl glycol.

The composition may comprise at least 10 wt.% of a humectant, wherein the humectant comprises polyol. For example, the humectant may be present in the composition in an amount by weight of at least 11%, at least 12%, at least 13%, at least 14%, or at least 15%. The humectant may be present in the compositions of the present invention in an amount from 10 wt.% to 22 wt.%, such as from 11 wt.% to 22 wt.%, or 12 wt.% to 22 wt.%, or 13 wt% to 22 wt.%, or 14 wt% to 22 wt.%, or from 10 wt.% to 21 wt%, or from 11 wt.% to 21 wt.%, or 12 wt.% to 21 wt%, 13 wt.% to 21 wt.%, or 14 wt.% to 21 wt.%, 10 wt.% to 20 wt.%, or 11 wt.% to 20 wt.%, or from 12 wt.% to 20 wt.%, or from 13 wt.% to 20 wt.%, or from 14 wt.% to 20 wt.%, or from 10 wt.% to 19 wt.%, or from 12 wt.% to 19 wt.%, or from 14 wt.% to 19 wt.%, or from 10 wt.% to 18 wt.%, or from 11 wt.% to 18 wt.%, or from 12 wt.% to 18 wt.%, or from 13 wt.% to 18 wt.%, or from 14 wt.% to 18 wt.%, or from 10 wt.% to 17 wt.%, wherein the percentages are based on the total weight of the composition.

The humectant in the composition of the present invention may comprise a polyol, wherein the polyol comprises or consists of glycerin. The skincare composition of the present invention may comprise from 10 to 22 wt.% (e.g. from 10 to 18 wt.%) of a humectant, wherein the humectant comprises or consist of glycerin.

The humectant in the composition of the present invention may comprise a polyol, wherein the polyol comprises glycerin, caprylyl glycol and/or ethylhexylglycerin. The polyol may comprise glycerin (e.g. 10 to 21 wt.% glycerin) and caprylyl glycol (e.g., from 0.1 to 0.9 wt.%, or from 0.1 to 0.6 wt.%, from 0.1 to 0.4 wt.%). The polyol may comprise glycerin, caprylyl glycol and ethylhexylglycerin. The composition may comprise glycerin, caprylyl glycol and ethylhexylglycerin, wherein the amount of caprylyl glycol and ethylhexylglycerin in the composition is from 0.1 to 0.9 wt.%, or from 0.1 to 0.6 wt.%, or from 0.1 to 0.4 wt.%, and wherein optionally the amount of glycerin in the composition is from 10 to 21% wt.%, or from 10 to 20% wt.%.

The humectant in the composition of the present invention may comprise a polyol, wherein the polyol comprises glycerin, saccharide isomerate, caprylyl glycol, ethylhexylglycerin or combinations thereof. The humectant in the composition of the present invention may comprise a polyol, wherein the polyol comprises glycerin (e.g. 10 to 21 wt.% glycerin) and saccharide isomerate, wherein the saccharide isomerate may be present in the composition in an amount by weight of from 0.05% to 1%, such as from 0.05% to 0.95%, or from 0.05% to 0.85%, or from 0.05% to 0.80%, or from 0.05% to 0.75%, from 0.05% to 0.7%, or from 0.05% to 0.65%, or from 0.05% to 0.6%, or from 0.05% to 0.55%, or from 0.05% to 0.5%, or from 0.1% to 0.85%, from 0.1% to 0.80%, or from 0.1% to 0.75%, or from 0.1% to 0.7%, or from 0.1% to 0.65%, or from 0.1% to 0.6%, or from 0.1% to 0.55%, or from 0.1% to 0.5%, or from 0.2% to 0.85%, from 0.2% to 0.80%, or from 0.2% to 0.75%, or from 0.2% to 0.7%, or from 0.2% to 0.65%, or from 0.2% to 0.6%, or from 0.2% to 0.55%, or from 0.2% to 0.5%, or from 0.4% to 0.85%, from 0.4% to 0.80%, or from 0.4% to 0.75%, or from 0.4% to 0.7%, or from 0.4% to 0.65%, or from 0.4% to 0.6%, or from 0.4% to 0.55%, or from 0.4% to 0.5%.

### Emulsions

The composition of the present invention may be aqueous or non-aqueous and comprise of a single-phase system or multiple phase system. The skincare composition may include but is not limited to liquids, gels, balms, oils or solids. Single or multiple phase compositions are envisaged. Multiple phase systems include but are not limited to microemulsions, emulsions, and products with discrete separate phases. The skincare composition of the present invention may be an emulsion. Emulsions include water-in-oil, oil-in-water emulsions and multiple emulsions (water in oil in water or oil in water in oil for example). Products with discrete separate phases include bi or triphasic systems where the individual water or oil phases can be visibly seen.

Where the skincare composition of the invention is an emulsion, it comprises an oil and a water phase.

The composition of the present invention may be an oil-in-water emulsion, for example a cationic oil-in-water emulsion. Oil in water emulsions are preferred by consumers as they have a lighter feel on the skin due to them being water based.

The oil in water emulsion may therefore comprise at least 40% by weight water, such as at least 45%, at least 50% or at least 90% by weight water. The oil in water emulsion of the present invention may comprise 90% by weight water or less, such as 85% or less, 80 % or less, or 75% by weight or less. The emulsion may comprise 50% to 95% by weight of the emulsion of water, such as from 50% to 90 % by weight, or from 50% to 85 % by weight, or from 50% to 80 % by weight, from 55% to 95 % by weight, or from 55% to 90 % by weight, or from 55% to 85 %, from 55% to 80 % by weight of water.

The water may comprise de-ionized water, purified water, natural spring water, rose water or the like. Mixtures of more than one of these may also be used. **In** one embodiment de-ionized or purified water may be used.

It will be appreciated that the water phase may comprise further components as part of a cosmetic emulsion. The water phase may comprise additional components such as pH adjusters, thickeners, active ingredients such as peptides or other anti-ageing ingredients, and combinations thereof.

The oil phase of an emulsion can be provided by any suitable oily component. Suitable oils for the oil phase may comprise for example: a) hydrocarbon oils, such as paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetyldimethicone; e) fatty acid esters and ethers, such as isopropyl palmitate or isopropyl myristate and polypropylene glycol-15 stearyl ether; f) fatty alcohols, such as brassica alcohol, behenyl alcohol, cetyl alcohol, cetearyl alcohol or stearyl alcohol; or g) mixtures thereof, for example, the blend of waxes available commercially under the trade name Cutina (BASF).

The emulsion may comprise at least 0.1 wt.% of an oil phase. For example, the emulsion may comprise at least 3 wt.% of oil phase, such as at least 5 wt.%, or at least 10 wt.%. The emulsion may comprise 0.1 wt.% to 55% wt.% of oil phase. In one embodiment, emulsion may comprise 0.1 wt.% to 50% wt.% of oil phase, more preferably from 0.1 wt.% to 40 wt.%, or from 0.1 wt.% to 30 wt.%, or from 1 wt.% to 55 wt.%, or from 1 wt.% to 50 wt.%, or from 1 wt.% to 40 wt.%, or from 1 wt.% to 30 wt.%, or from 3 wt.% to 55 wt.%, or from 3 wt.% to 50 wt.%, or from 3 wt.% to 40 wt.%, or from 3 wt.% to 30 wt.%, or from 5 wt.% to 55 wt.%, or from 5 wt.% to 50 wt.%, or from 5 wt.% to 40 wt.%, or from 5 wt.% to 30 wt.%, or from 5 wt.% to 20 wt.%, or from 5 wt.% to 10 wt.% of oil phase.

The oil phase of the emulsion may comprise a combination of oil, wax or butter. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are a type of suitable wax based on alkylmethylsiloxane. The wax may be fatty alcohol wax (e.g. cetearyl alcohol). Oils are typically lipophilic and liquid at room temperature with lower molecular weights than waxes. Where present, wax or butter may be present at up to 30% by weight of the oil phase of the emulsion. The oil phase may contain wax or butter at levels of up to 20% by weight of the oil phase of the emulsion. The oil phase may contain wax or butter at levels of up to 10% by weight of the oil phase of the emulsion, such as from 1 to 10%, or from 1 to 8% by weight of the oil phase of the emulsion. The waxes may comprise cocoa butter, shea butter or cetearyl alcohol.

The composition of the present invention may be in the form of an oil-in-water emulsion wherein the oil phase of the emulsion may comprise a fatty alcohol wax (e.g. Cetearyl alcohol).

The oil phase may comprise silicone oils which are silicone elastomers. Suitable for use herein are silicone elastomers which can be emulsifying or non-emulsifying crosslinked siloxane elastomers or combinations thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon- bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester. Preferred silicone elastomers include dimethicone crosspolymer and Polysilicone-11.

The oil phase of the emulsion may comprise a triglyceride. The triglyceride may be a C3 to C16 triglyceride for example a C6 to C12 triglyceride. Examples include tripalmitin and caprylic/capric triglyceride. In one example the triglyceride may be caprylic/capric triglyceride.

### Other components

The skincare composition may comprise only cosmetically acceptable components. The skilled person is able to identify cosmetically acceptable components, and these are well known in the field of the invention.

In some embodiments, the composition may further comprise one or more antioxidant agents selected from a) ascorbic acid its salts, esters, glucosides and glucosamines, particularly sodium ascorbyl phosphate, magnesium ascorbyl phosphate, ascorbyl palmitate and ethyl ascorbic acid b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts (not including Annona, ginseng and Trifolium extracts), particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica^{™} by Merck Speciality chemicals, and Grape Seed oil, such as that available from Chesham Chemicals Limited.

The composition may comprise one or more antioxidant agents selected from green tea extract (e.g. Camellia sinensis leaf extract), Quercus petraea fruit extract (such as that sold under the trade name of Phytessence French Oak^{™} from Croda) and ferulic acid (also known as hydroxy cinnamic acid).

The composition may further comprise one or more antioxidant agents selected from flavonoid species, including flavones, flavonols, flavanones, flavanols, anthrocyanidins and isoflavonoids; phenoic acid species; stilbenes; lignans or combinations thereof, which provides an antioxidant benefit. The antioxidant agent may also be a plant extract or derivative thereof. Plants provide a rich and cheap source of antioxidant agents and are therefore an efficient source of said agents. Similar actives can be also prepared synthetically and as such are analogues. The term "antioxidant agent" is also intended to cover said analogues. The plant extract may be selected from the group consisting of essential oils, extracts from leaves, extracts from stems, extracts from petals, extracts from seeds, extracts from roots, extracts from pollen, and combinations thereof.

The antioxidant agent(s) may be selected from the group consisting of dimethylmethoxy chromanol (such as that sold under the trade name of LIPOCHROM AN^{®} Molecule from Lipotec), Myrtus communis leaf extract (such as that sold under the trade name of DEXOSINE BIO^{®} from Silab), Camellia japonica extract (e.g., a Camellia japonica flower extract such as that sold under the trade name of RedSnow^{®} from Clariant. The extract may be obtained from the leaves of Camellia japonica. The extract may be obtained from the roots of Camellia japonica. Camellia japonica flowers may be collected on Jeju island and may contain Protocatechuic acid (200ppm)), emblica extract (e.g. Phyllanthus emblica fruit extract (also known as Emblica officinalis (Gooseberry) fruit extract)), ginkgo extract (e.g. Ginkgo biloba leaf extract), Argania spinosa leaf extract (such as that sold under the trade name of Arganyl^{™} from BASF), Pinus pinaster bark extract (such as that sold under the trade name of Pycnogenol), Resveratrol (such as that sold under the trade name of REGU^{®}-FADE), and combinations thereof.

The amounts of the antioxidant agents used in the skincare compositions are expressed as dry weights, as understood by a man skilled in the art. The total amount of antioxidant agents optionally present in the skincare compositions may range from 0.005% to 10% by weight, preferably 0.01% to 5%, most preferably 0.05% to 1.0% by weight of the total weight of the skincare composition.

The compositions of the invention may further comprise one or more ceramides. Ceramides are a family of waxy lipid molecules. A ceramide is composed of sphingosine and a fatty acid.

The composition comprises at least one ceramide. The at least one ceramide may comprise ceramide 1 (ceramide EOP), ceramide 2 (ceramide NG), ceramide 3 (ceramide NP) and/or ceramide 6 (ceramide AP). The composition preferably comprises ceramide 3 (ceramide NP), also known as N-oloeoyl-4-hydroxysphinganine. The composition preferably comprises ceramide 6 (ceramide AP).

The composition may comprise at least two ceramides, such as ceramide 3 and ceramide 6. The composition may comprise at least 3 ceramides, such as ceramide 1, ceramide 3, and ceramide 6.

The compositions of the invention may optionally comprise a skin conditioning agent. Said skin conditioning agents may preferably be selected from the group consisting of emollients, moisturisers, or mixtures thereof. Where present, they are preferably present at a level of from 0.01% to 20%, more preferably from 0.1% to 10%, most preferably from 0.5% to 7% by weight of the skincare composition.

Preferred skin conditioning agents are selected from the group consisting of guanidine, urea, and glycolate salts, salicylic acid, lactic acid and lactate salts, aloe vera, shea butter, gluconodeltalactone, and starches and their derivatives, pyrrolidone, carboxylic acid, lactamide monoethanolamine, acetamide monoethanolamine, allantoin and mixtures thereof.

The compositions of the invention may comprise one or more vitamins. The composition of the present invention may comprise vitamin B (e.g., vitamin B1 to vitamin B12) or a vitamin B derivative (e.g., a derivative of vitamin B1 to B12, such as a vitamin B3). The composition of the present invention may comprise a vitamin B3 compound, such as niacinamide. Vitamin B3 and niacin, are the common names for nicotinic acid. The physiologically active form of niacin is niacinamide. Niacin and niacinamide (nicotinamide or nicotinic acid amide) function in the body as a component of two coenzymes: nicotinamide adenine dinucleotide (NAD/NAD+) and nicotinamide adenine dinucleotide phosphate (NADP).

The compositions of the present invention preferably may comprise one or more vitamin B3 compounds. Preferred vitamin B3 compounds are niacinamide and tocopherol nicotinate. Niacinamide is more preferred. Salts of the vitamin B3 compound are also useful herein. Non-limiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1-C18 carboxylic acid salts, e.g., acetate, salicylate, glycolate, lactate, malate, citrate, preferably monocarboxylic acid salts such as acetate). The vitamin B3 compound may be included as the substantially pure material, or as an extract obtained by suitable physical and/or chemical isolation from natural sources. The vitamin B3 compound is preferably substantially pure, more preferably essentially pure.

The Vitamin B or vitamin B derivative (e.g. vitamin B3 compound, such as niacinamide) may be present in the composition in an amount by weight of at least 0.01%, at least 0.05%, at least 0.1%, at least 0.5%, at least 1%, at least 2%, at least 3%, at least 4%; and/or the vitamin B or vitamin B derivative (e.g., vitamin B3 compound such as niacinamide) may be present in the composition in an amount by weight of 5% or less, 4% or less, 3% or less, 2% or less, 1% or less, 0.5% or less, 0.1% or less, or 0.05% or less. Where more than one vitamin B/vitamin B derivative is present, the values apply to the total amount of vitamin B or vitamin B derivative.

In particular, the vitamin B or vitamin B derivative (e.g., vitamin B3 compound such as niacinamide) may be present in the composition in an amount by weight of from 0.1 to 5%, such as from 0.1 to 4%, or from 0.1 to 3.5%, or from 0.1 to 3%, or from 0.1 to 2.5%, or from 0.1 to 2%, or form 0.5 to 4%, or from 0.5 to 3.5%, or from 0.5 to 3%, or from 0.5 to 2.5%, or form 1 to 4%, or from 1 to 3.5%, or from 1 to 3%, or from 1 to 2.5%, or form 1.5 to 4%, or from 1.5 to 3.5%, or from 1.5 to 3%, or from 1.5 to 2.5%, or from 1.5 to 2%.

The composition of the present invention may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid.

In one embodiment the skincare compositions may comprise vitamin K, vitamin K derivatives, vitamin H, vitamin D, vitamin D derivatives and mixtures thereof. In an alternative embodiment of the present the skincare compositions comprise vitamin E, vitamin E derivatives such as tocopherol and tocopheryl acetate, and provitamins thereof, such as panthenol and mixtures thereof.

In a further embodiment, the skincare compositions of the invention comprise retinoid compounds, including retinoic acid, retinaldehyde, retinol and derivatives thereof. In one embodiment the skincare compositions comprise retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e.g. plant) sources. In one embodiment, when vitamin compounds are present in the skincare compositions of the instant invention, the skincare compositions comprise from about 0.0001% to 50%, more preferably from 0.001% to 10%, still more preferably from 0.01% to 8%, and still more preferably from 0.1% to 5%, by weight of the skincare composition, of the vitamin compound.

The compositions of the invention may optionally comprise a sunscreen component. The sunscreen may comprise organic or inorganic sun filters or a combination of the two. Suitable inorganic sun filters include those selected from the group consisting of microfine titanium dioxide, microfine zinc oxide, boron nitride and mixtures thereof. Suitable organic sunscreens include those selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4-(tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino-(p-carbo-2-ethyl-hexyl-1-oxi)-1, 3,5-triazine, h) camphor derivatives such as methylbenzylidene camphor and i) mixtures thereof. Other preferred sunscreen ingredients include those selected from the group consisting of homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreen agent is optionally present in an amount from 0.1 to 10% by weight of the skincare composition.

The composition of the present invention may comprise a peptide. Peptides are defined as compounds comprising an uninterrupted sequence of amino acids. Preferred peptides are selected from the group consisting of dipeptides, tripeptides, tetrapeptides, pentapeptides and mixtures thereof.

The peptide may comprise synthetic peptide that inhibits the enzymes plasmin and urokinase.

The peptide may comprise an amidino benzylamine derivative. The peptide may be selected from amidinobenzyl benzylsulfonyl D-seryl homophenylalaninamide acetate (ABSHA), benzylsulfonyl-D-Ser-lys-(4-amidino-benzylamide), benzylsulfonyl-D-Ser-Gly-4-amidino-benzylamide and benzylsulfonyl-D-Ser-Ala-4- amidino-benzylamide. The peptide may comprise or consist of amidinobenzyl benzylsulfonyl D-seryl homophenylalaninamide acetate. The peptide may be commercially available under trade name SYN-UP^{®} from DSM Nutritional Products Ltd.

The peptide (e.g., ABSHA) may be present in the composition in an amount by weight of from 0.0001 to 0.0004% (e.g., from 1ppm to 4ppm), such as from 0.00015 to 0.0004%, or from 0.0002 to 0.0004%, or from 0.00025 to 0.0004%, or from 0.0001 to 0.00035%, or from 0.00015 to 0.00035%, or from 0.0002 to 0.00035%, or from 0.00025 to 0.00035%, or from 0.0001 to 0.0003%, or from 0.00015 to 0.0003%, or from 0.0002 to 0.0003%, or from 0.00025 to 0.0003%, or from 0.0001 to 0.00025%, or from 0.00015 to 0.00025%, or from 0.0002 to 0.00025%.

The skincare compositions of the invention may also optionally comprise one or more of the following optional ingredients. The skincare compositions of the invention may comprise preservatives such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, sodium benzoate, chlorhexidine digluconate, isothiazolone and sodium propyl paraben, suitably in an amount of from 0.01% to 10% by weight of the skincare composition.

The skincare compositions of the invention may comprise thickeners, viscosity modifying agents and/or gelling agents, such as acrylic acid polymers e.g., available commercially under the trade name Carbopol, Ultrez or Novethix (Lubrizol) or Sepigel, Sepiplus and Simulgel (Seppic). Modified celluloses may also be added, for example hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name "Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcohols, fatty alcohols and alkyl galactomannans available under the trade name N-Hance from Hercules, suitably in an amount of from 0.1% to 10% by weight of the skincare composition.

Sequestering agents may be added to the skincare compositions, such as ethylenediamine tetraacetic acid and salts thereof, suitably in an amount of from 0.005% to 0.5% by weight of the skincare composition.

The skincare compositions may also comprise suitable, cosmetically acceptable diluents, carriers and/or propellants such as dimethyl ether.

The skincare compositions may also include pearlising agents such as stearic monoethanolamide and/or mica, suitably in an amount of from 0.01% to 10% by weight of the skincare composition.

The skincare compositions may include perfumes. In some embodiments the perfumes are present in an amount of from 0.01% to 2% by weight of the skincare compositions, as may water soluble dyes such as tartrazine, suitably in an amount of from a trace amount (such as 1 x 10-5 %) to 0.1 % by weight of the skincare composition.

The skincare compositions may also include pH adjusting agents such as sodium hydroxide, aminomethyl propanol, calcium gluconate, aspartic acid, triethanolamine, suitably in an amount of from 0.01 % to 10% by weight of the skincare composition. The skincare compositions may be buffered by means well known in the art, for example by use of buffer systems comprising succinic acid, citric acid, lactic acid, and acceptable salts thereof, phosphoric acid, mono-or disodium phosphate and sodium carbonate. Suitably, the skincare composition may have a pH between 3 and 10, preferably between 4 and 8, more preferably from 4.5 to 6.

### Methods and processes

The methods of the present invention may comprise applying on a person's skin the skincare compositions described in the present invention.

The present invention also relates to a method comprising applying the skincare compositions according to the present invention to the skin of a subject.

The invention also relates to a cosmetic method of moisturising or hydrating a skin in need thereof, the method comprising topically administering the skincare composition according to the present invention to the skin of a subject.

The present invention also provides a cosmetic method of reducing skin dryness, the method comprising topically administering the skincare composition according to the present invention to the skin of a subject.

The invention further concerns a cosmetic method for improving the skin barrier function, the method comprising topically administering the skincare compositions according to the present invention to the skin of a subject.

The invention further relates to a method of reducing transepidermal water loss (TEWL) from the skin's surface, the method comprising the topical application of the skincare composition of the present invention to the skin of a subject.

The invention further relates to a method of improving the radiance of the skin and/or improving the healthy visual appearance of the skin, the method comprising the topical application of the skincare composition according to the present invention to the skin of a subject.

### Definitions

Unless otherwise indicated, a reference to the composition of the invention is a reference to the compositions of all aspects and embodiments of the invention.

All percentages provided herein are percentages by weight of the composition unless otherwise specified.

### Description of the Drawings

### Examples

The present invention is further described by the following examples.

### Exemplary Formulations:

In the following exemplary formulations (examples 1-3), the active levels of components are shown. Other components as discussed in the description may be selected by the skilled person such that the formulation totals 100wt.%.

### Example 1 - Cationic moisturiser (Distearoylisopropyl dimonium methosulfate)

| Material | wt.% |
|---|---|
| Glycerin | 10.00 |
| Water | 64.40 |
| Phenoxyethanol | 0.40 |
| Caprylyl glycol & Ethylhexylglycerin | 0.20 |
| Dimethicone | 1.00 |
| Caprylic/capric triglyceride | 10.00 |
| Butyrospermum parkii (Shea) butter | 2.00 |
| Distearoylisopropyl dimonium methosulfate | 5.00 |
| Glyceryl stearate | 1.00 |
| Cetearyl alcohol | 4.00 |
| Saccharide isomerate & Water & Citric acid & Sodium citrate | 0.50 |
| Niacinamide | 1.00 |
| Amidinobenzyl benzylsulfonyl D-seryl homophenylalaninamide acetate & Glycerin & Water & Citric acid & Sodium hydroxide | 0.50 |

### Method of manufacture

1. In the main vessel add water, glycerin, saccharide isomerate and water and citric acid and sodium citrate and Niacinamide. Stir until solids dissolved and heat to 70-75°C.
2. In a separate vessel weigh out the oil phase (dimethicone, caprylic/capric triglyceride, butyrospermum parkii (Shea) butter, distearoylisopropyl dimonium methosulfate, glyceryl stearate and cetearyl alcohol). Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in phenoxyethanol, caprylyl glycol and ethylhexylglycerin and amidinobenzyl benzylsulfonyl D-seryl homophenylalaninamide acetate and glycerin and water and citric acid and sodium hydroxide.
6. Dissolve Niacinamide in a small amount of water and stir in.
7. Continue to the cool the product to room temperature.
8. Make to weight with water and stir smooth.

### Example 2 - Cationic moisturiser (Brassicyl valinate esylate)

| Material | wt.% |
|---|---|
| Glycerin | 12.00 |
| Water | 60.60 |
| Phenoxyethanol | 0.40 |
| Caprylyl glycol & Ethylhexylglycerin | 0.20 |
| Dimethicone | 1.00 |
| Caprylic/capric triglyceride | 10.00 |
| Butyrospermum parkii (Shea) butter | 2.00 |
| Brassica alcohol & Brassicyl valinate esylate & Brassica glycerides | 6.00 |
| Calcium gluconate | 0.80 |
| Cetearyl alcohol | 4.00 |
| Niacinamide | 3.00 |

### Method of manufacture

1. In the main vessel add water, glycerin and calcium gluconate. Stir until solids dissolved and heat to 70-75°C.
2. In a separate vessel weigh out the oil phase (dimethicone, caprylic/capric triglyceride, butyrospermum parkii (Shea) butter, bisabolol, brassica alcohol and brassicyl valinate esylate, and brassica glycerides, glyceryl stearate and cetearyl alcohol). Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in phenoxyethanol and caprylyl glycol and ethylhexylglycerin.
6. Dissolve Niacinamide in a small amount of water and stir in.
7. Continue to the cool the product to room temperature.
8. Make to weight with water and stir smooth.

### Example 3 - Cationic moisturiser (Brassicamidopropyl dimethylamine)

| Material | wt.% |
|---|---|
| Glycerin | 15.00 |
| Water | 60.52 |
| Phenoxyethanol | 0.40 |
| Caprylyl glycol & Ethylhexylglycerin | 0.20 |
| Dimethicone | 1.00 |
| Caprylic/capric triglyceride | 10.00 |
| Butyrospermum parkii (Shea) butter | 2.00 |
| Brassicamidopropyl dimethylamine | 1.00 |
| Aspartic acid | 0.38 |
| Cetearyl alcohol | 3.00 |
| Glyceryl stearate | 1.50 |
| Saccharide isomerate & Water & Citric acid & Sodium citrate | 0.50 |
| Niacinamide | 4.00 |
| Amidinobenzyl benzylsulfonyl D-seryl homophenylalaninamide acetate & Glycerin & Water & Citric acid & Sodium hydroxide | 0.50 |

### Method of manufacture

1. In the main vessel add water, glycerin, saccharide isomerate and water and citric acid and sodium citrate, aspartic acid and Niacinamide. Stir until solids dissolved and heat to 70-75°C.
2. In a separate vessel weigh out the oil phase (dimethicone, caprylic/capric triglyceride, butyrospermum parkii (Shea) butter, brassica alcohol and brassicyl valinate esylate, and brassica glycerides, glyceryl stearate and cetearyl alcohol). Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in phenoxyethanol, caprylyl glycol and ethylhexylglycerin and amidinobenzyl benzylsulfonyl D-seryl homophenylalaninamide acetate and glycerin and water and citric acid and sodium hydroxide.
6. Continue to the cool the product to room temperature.
7. Make to weight with water and stir smooth.

### Example 4 - Corneometer study over 4 hours

The effects of the composition of the invention on moisture in the skin was evaluated by Corneometry. The measuring principle of the Corneometer is based on capacitance measurement of a dielectric medium (the ability of a body to hold charge).

Studies were carried out using the Multiprobe Adapter MPA5 and Corneometer CM825 Probes. The measurement depth was the first 10-20 µm of the stratum corneum. The probe was wiped (with a dry tissue) between each measurement to ensure any residual moisture left on the probe does not affect future measurements, care was taken to apply the probe to the skin with even pressure and subjects were allowed to acclimatise in the climate controlled area for at least 30 minutes before measurements are taken.

2mg/cm2 of the compositions (A-D) were applied in a 2cm x 2cm area of the skin and assessed at 4 hours. Six measurements were taken at each site for each timepoint, ensuring that the probe head is wiped gently with a tissue between each individual measure. The difference in reading from T0 to TX for the untreated control sites was compared to the difference in readings from T0 to TX for each test product at each timepoint. This determines if the test product has had a significant effect on the hydration of the skin over time, or if the changes in hydration are due to natural fluctuations in the skin. The normality of the data was checked using the Stats Package (DOC-00360 Use of D121 Stat Package Statistical Application) and if the data is not normal a Wilcoxon Signed Rank Test was used to test for statistical significance instead of the Student t test.

Compositions A-D were tested:
(A) Comparative cationic moisturising cream containing behentrimonium methosulfate (a quaternary ammonium salt emulsifier) and 8.3% glycerin.
(B) Comparative cationic moisturising cream containing distearyldimonium chloride (a quaternary ammonium salt emulsifier) 12.9 % glycerin.
(C) Cationic moisturising cream according to the present invention containing brassicamidopropyl dimethylamine, niacinamide, saccharide isomerate, amidinobenzyl benzylsulfonyl D-seryl homophenylalaninamide acetate and 15% glycerin.
(D) Comparative non-ionic moisturiser containing 8% glycerin, 1% propanediol, 0.75% panthenol and 1% saccharide isomerate.

Figure 1 shows the % change in skin moisturisation measured using a Corneometer after 4 hours of application compositions A-D on the skin. The results show that composition (C) according to the invention provides better moisturisation than moisturisers containing traditional quat emulsifiers (A and B) and compositions without a cationic emulsifier (D).

### Example 5- Consumer testing

A consumer testing of a composition according to the present invention containing cationic emulsifier brassicyl valinate esylate and 12 wt.% glycerin was carried out. This included 150 volunteers.

Following a four weeks trial the following results were obtained:
- 84% felt the product left the skin looking moisturised for longer through the day.
- 85% felt the product left the skin feeling more moisturised for longer through the day.
- 75% felt the skin was improved after 4 weeks of using the product.

## Claims

1. A skincare composition comprising (a) cationic emulsifier; and (b) at least 10 wt.% of a humectant, wherein the humectant comprises polyol.

2. The composition according to claim 1, wherein the cationic emulsifier comprises an ester quaternary ammonium cationic emulsifier and/or a non-quaternary ammonium cationic emulsifier.

3. The composition according to claim 1 or claim 2, wherein the cationic emulsifier is present in the compositions in an amount of from 0.05% to 14% by weight.

4. The composition according to any one of claims 1 to 3, wherein the cationic emulsifier comprises an ester quaternary ammonium cationic emulsifier, a cationic amino lipid emulsifier, an amidoamine cationic emulsifier or combinations thereof

5. The composition according to any one of claims 1 to 4, wherein the cationic emulsifier comprises distearoylisopropyl dimonium methosulfate, brassicamidopropyl dimethylamine, brassicyl valinate esylate, distearoylethyl dimonium chloride, bis-(isostearoyl/oleoyl isopropyl) dimonium methosulfate, cannabisamidopropyl dimethylamine, stearamidopropyl dimethylamine, brassicyl isoleucinate esylate or combinations thereof.

6. The composition according to any one of claims 1 to 5, wherein the cationic emulsifier comprises:
a) an ester quaternary ammonium emulsifier which is present in the composition in an amount by weight of from 1 to 6%, wherein optionally the ester quaternary ammonium compound comprises distearoylisopropyl dimonium methosulfate; or
b) a non-quaternary ammonium cationic emulsifier, which is present in the composition in an amount by weight from 0.05 to 14%.

7. The composition according to any one of claims 1 to 6, wherein the amount of humectant in the composition is from 10 to 22 wt.%.

8. The composition according to any one of claims 1 to 7, wherein the polyol comprises glycerin.

9. The composition according to any one of claims 1 to 8, wherein one or more of the following applies:
a) the polyol comprises glycerin, caprylyl glycol, ethylhexylglycerin, saccharide isomerate or combinations thereof;
b) the polyol comprises glycerin and saccharide isomerate;
c) the polyol comprises glycerin, caprylyl glycol and ethylhexylglycerin;
d) the polyol comprises glycerin, caprylyl glycol, ethylhexylglycerin and saccharide isomerate;
e) the composition is an emulsion; and
f) the composition is in the form of an oil-in-water emulsion.

10. Use of the skincare composition according to any one of claims 1 to 9, for the topical cosmetic application to the skin of a subject.

11. Use of the skincare composition according to any one of claims 1 to 9 for moisturising and/or hydrating the skin of a subject.

12. Use of a composition according to any one of claims 1 to 9 on skin as a non-therapeutic cosmetic treatment for any one or more of the following: improving skin moisture content, hydrating skin, improving the radiance of the skin, improving skin suppleness, reducing skin tightness, improving the healthy visual appearance of the skin, reducing skin dryness, improving the skin barrier function or any combination thereof.

13. A method comprising applying the skincare composition according to any one of claims 1 to 9 to the skin of a subject.

14. A cosmetic method for moisturising or hydrating a skin in need thereof, the method comprising topically administering the skincare composition according to any one of claims 1 to 9 to the skin of a subject.

15. A cosmetic method of reducing skin dryness, the method comprising topically administering the skincare composition according to any one of claims 1 to 9 to the skin of a subject.
